# EUROPÄISCHE PATENTSCHRIFT

(11) **EP 0 670 837 B1**
(45) Veröffentlichungstag und Bekanntmachung des Hinweises auf die Patenterteilung: **16.10.1996**
(21) Anmeldenummer: 94900718.1
(22) Anmeldetag: 22.11.1993
(51) Int. Cl.: C07D 471/04, A61K 31/44

(54) **NEUES VERFAHREN ZUR HERSTELLUNG VON BETA-CARBOLINEN**
METHOD OF PREPARING BETA-CARBOLINES
NOUVEAU PROCEDE DE PREPARATION DE BETA-CARBOLINES

(30) Priorität: 24.11.1992 DE 4240672
(43) Veröffentlichungstag der Anmeldung: 13.09.1995
(73) Patentinhaber: SCHERING AKTIENGESELLSCHAFT, 13353 Berlin (DE)
(72) Erfinder: HAFFER, Gregor, D-12207 Berlin (DE); NICKISCH, Klaus, D-12307 Berlin (DE)
(86) Internationale Anmeldenummer: DE9301116
(87) Internationale Veröffentlichungsnummer: WO9412498

(56) Entgegenhaltungen:
- EP-A- 0 190 987
- WO-A-92/05269
- US-A- 4 877 792

## Beschreibung

Die Erfindung betrifft ein neues Verfahren zur Herstellung von β-Carbolinen durch Dehydrierung von Tetrahydro-β-carbolinen mit Trichlorisocyanursäure.

Auf Grund ihrer Affinität an die Benzodiazepin-Rezeptoren zeigen β-Carboline Wirkungen auf das Zentralnervensystem und eignen sich zur Herstellung von Arzneimitteln. Die großtechnische Herstellung von β-Carbolinen im Betrieb hat daher in letzter Zeit großes Interesse gefunden. Die Dehydrierung von 1,2,3,4-Tetrahydro-β-carbolinderivaten kann durch Fermentation mit einer Pilzkultur erfolgen (WO-A-9205 269). Die Synthese der Wirkstoffe erfolgt über mehrere Stufen, wobei die Ausbeuten in den einzelnen Stufen von der Reinheit des Ausgangsproduktes beeinflußt werden. So ist beispielsweise aus EP-239667 bekannt, daß β-Carboline über 3-Stufen darstellbar sind, wobei in der letzten Stufe eine Aromatisierung des C-Ringes vorgenommen wird. Die als Ausgangsprodukte für die Dehydrierung eingesetzten 1,2,3,4,-Tetrahydro-β-Carboline bzw. deren 1-Carbonsäurederivate werden isoliert und nach EP-190 987 mit tert. Butylhypochlorit in Gegenwart von Basen umgesetzt. Tert. Butylhypochlorit ist für großtechnische Synthesen schlecht geeignet, da es sich bei erhöhter Temperatur und bei der Einwirkung von Licht exotherm in Chlormethan und Aceton zersetzen kann. Nicht nur das Gefährdungspotential sondern auch die schlechtere Dosierbarkeit von tert. Butylhypochlorit erfordern dessen Austausch in großtechnischen Synthesen.

Es ist daher wünschenswert, ein Verfahren zur Herstellung von β-Carbolinen zu entwickeln, das die Aufarbeitung von Zwischenstufen nicht erfordert und mit gut zugänglichen und gut lagerbaren Substanzen die großtechnische Herstellung der Verbindungen ermöglicht.

Überraschenderweise wurde nun gefunden, daß bei der Dehydrierung von Tetrahydro-β-carbolinen mit Trichlorisocyanursäure, die in sehr guten Ausbeuten abläuft, eine Aufarbeitung und Isolierung der Zwischenprodukte nicht erforderlich ist. Das erfindungsgemäße Verfahren ermöglicht, daß β-Carboline ausgehend von Pseudograminderivaten und Schiffschen Basen in einer Eintopfreaktion herstellbar sind. Ein weiterer Vorteil des erfindungsgemäßen Verfahrens besteht darin, daß Trichlorisocyanursäure lagerstabil und als Feststoff gut dosierbar ist und bei der Aufarbeitung des Reaktionsgemisches mit wässrigen Basen die wasserlöslichen Umsetzungsprodukte leicht abtrennbar sind.

Die Erfindung betrifft das Verfahren zur Herstellung von Verbindungen der Formel I 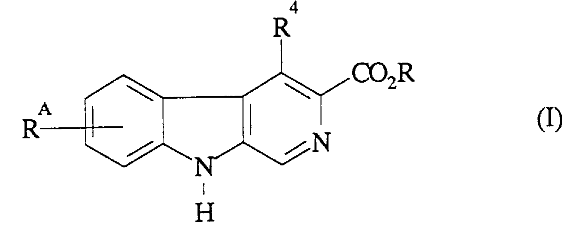 worin
- R^{A}: Wasserstoff, Halogen, -CHR¹-R², gegebenenfalls mit Halogen, C₁₋₄-Alkoxy oder Amino substituiertes Phenyl, OR⁵ oder einen 5- oder 6-gliedrigen Hetarylrest mit 1-3 Stickstoff-, Sauerstoff - und/oder Schwefelatomen bedeutet und ein- bis zweifach stehen kann und
- R¹: Wasserstoff oder C₁₋₄-Alkyl,
- R²: Wasserstoff, C₁₋₄-Alkyl, -O-C₁₋₄-Alkyl oder einen gegebenenfalls ein- bis dreifach mit Halogen, Nitro, Cyano, C₁₋₄-Alkyl, C₁₋₄-Alkoxy, Amino, C₁₋₄-Alkoxycarbonyl, C₁₋₄-Alkylthio oder Trifluormethyl substituierten Phenyl-, Benzyl- oder Phenoxy-Rest,
- R⁵: Wasserstoff, C₁₋₆-Alkyl, C₃₋₇-Cycloalkyl, einen gegebenenfalls ein- bis dreifach mit Halogen, Nitro, Cyano, C₁₋₄-Alkyl, C₁₋₄-Alkoxy, Amino, C₁₋₄-Alkoxycarbonyl, C₁₋₄-Alkylthio oder Trifluormethyl substituierten Phenyl-, Benzyl- -oder 5- oder 6-gliedrigen Hetarylrest mit 1-3 Stickstoff-, Sauerstoff- und/oder Schwefelatomen oder einen gegebenenfalls ein bis dreifach mit Halogen, Nitro, Cyano, C₁₋₄-Alkyl, C₁₋₄-Alkoxy, Amino, C₁₋₄-Alkoxycarbonyl, C₁₋₄-Alkylthio oder Trifluormethyl substituierten benzokondensierten Hetarylrest mit 1-2 Stickstoffatomen bedeutet,
- R⁴: Wasserstoff, C₁₋₄-Alkyl oder C₁₋₄-Alkoxy-C₁₋₂-alkyl und
- R: C₁₋₆-Alkyl bedeutet.,
dadurch gekennzeichnet, daß man eine Verbindung der Formel II 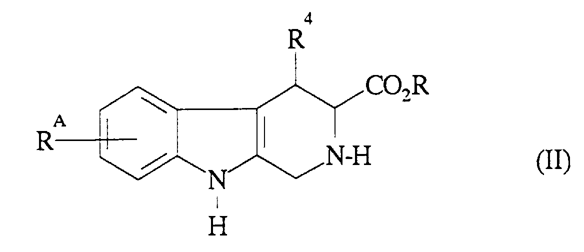 worin R, R^{A} und R⁴ die obige Bedeutung haben mit Trichlorisocyanursäure in Gegenwart von Basen aromatisiert.

Der Substituent R^{A} kann im A-Ring in Position 5-8, bevorzugt in 5-, 6- oder 7-Stellung stehen.

Alkyl beinhaltet jeweils sowohl gerad- als auch verzweigt-kettige Reste wie beispielsweise Methyl, Ethyl, Propyl, Isopropyl, Butyl, Isobutyl, sek. Butyl, tert. Butyl, Pentyl, Isopentyl und Hexyl.

Cycloalkyl kann jeweils für Cyclopropyl, Cyclobutyl, Cyclopentyl, Cyclohexyl, Cycloheptyl und 2-Methyl-cyclopropyl stehen, wobei 3-5 Kohlenstoffatome bevorzugt sind.

Bedeutet R⁵ oder R^{A} einen Hetarylrest, so ist dieser 5- oder 6-gliedrig und enthält 1-3 Heteroatome wie Stickstoff, Sauerstoff- und/oder Schwefel. Beispielsweise seien die folgenden 5- und 6-Ring Heteroaromaten genannt: Pyridin, Pyrimidin, Pyrazin, Pyridazin, Furan, Thiophen, Pyrrol, Thiazol, Imidazol, Triazin.

Bedeutet R⁵ einen benzokondensierten Hetarylrest, so enthält dieser vorzugsweise 1-2 Stickstoffatome wie Chinolin, Isochinolin, Chinoxalin oder Benzimidazol.

Der Substituent des Phenyl-, Benzyl-, Hetaryl- und benzokondensierten Hetarylrestes R⁵ kann ein- bis dreifach in jeder beliebigen Position stehen. Geeignete Substituenten sind Halogene, Nitro, Cyano, C₁₋₄-Alkyl, C₁₋₄-Alkoxy, Amino, C₁₋₄-Alkoxycarbonyl, C₁₋₄-Alkylthio und Trifluormethyl, wobei für den Phenyl- und Benzylrest die ein- bis zweifache Substitution mit Halogen bevorzugt ist.

Unter Halogen ist jeweils Fluor, Chlor, Brom oder Jod zu verstehen.

Als bevorzugte Hetarylrest und benzokondensierte Hetarylreste R⁵ sind stickstoffhaltige Heterocyclen zu betrachten, die gegebenenfalls ein- bis zweifach mit Halogen, C₁₋₄-Alkyl, C₁₋₄-Alkoxy oder Trifluormethyl substituiert sind, insbesondere mit Halogen.

Als Substituenten des Phenyl-, Benzyl- und Phenoxy-Restes R² sind die für R⁵ genannten Substituenten der Aromaten geeignet, insbesondere Halogen wie Chlor und Brom.

Die erfindungsgemäße Umsetzung erfolgt in inerten aprotischen Lösungsmitteln wie Kohlenwasserstoffen, halogenierten Kohlenwasserstoffen, cyclischen oder acyclischen Ethem und Estern wie beispielsweise Toluol, Benzol, Xylol, Hexan, Methylenchlorid, Chloroform, Dichlorethan, Tetrachlorkohlenstoff, Diethylether, Dimethoxymethan, Methyl-tert.-butylether, Isopropylacetat, Essigester, Dimethylformamid.

Als Basen sind alle organischen Basen geeignet insbesondere tertiäre Amine wie Triethylamin, Ethyldiisopropylamin, Pyridin, 1,5-Diazabicyclo-[4,3,0]-non-5-en (DBN), 1,8-Diazabicyclo-[5,4,0]-undec-7-en (DBU) und Dimethylaminopyridin (DMAP).

Die erfindungsgemäß verwendete Trichlorisocyanursäure wird in equivalenten Mengen oder in geringem Überschuß bezogen auf Chlor zugegeben. Im allgemeinen können 0,7-0,9 Molequivalente Trichlorisocyanursäure zugesetzt werden.

Die Reaktion wird bei Temperaturen von -20 °C bis zu Raumtemperatur durchgeführt, wobei durch Erhitzen des Reaktionsgemisches die Reaktionszeit verkürzt werden kann.

Zur Aufarbeitung des Reaktionsgemisches wird mit Wasser versetzt und die ausgefallenen Verbindungen der Formel I abgetrennt.

Die nach dem erfindungsgemäßen Verfahren hergestellten Verbindungen sind selbst wertvolle Pharmaka oder können als Zwischenverbindungen zur Herstellung von Wirkstoffen verwendet werden.

Die Herstellung der Ausgangsverbindungen ist bekannt oder kann nach bekannten oder hier beschriebenen Verfahren erfolgen. Beispielsweise wird die Herstellung der Pseudogramine in WO 91/16304 beschrieben.

### Beispiel 1

### A.) Herstellung von 2-(4-Methoxybenzyliden-amino)-essigsäureisopropylester

35,9 ml 4-Methoxybenzaldehyd werden unter Stickstoff und Rühren in 37,0 ml Dimethylformamid gelöst. Nach Zugabe von 53,8 ml Triethylamin und 21,0 g Magnesiumsulfat erwärmt man auf + 35 °C Innentemperatur, zu dieser Lösung werden 45,38 g Glycinisopropylesterhydrochlorid in 150 ml Dimethylformamid gelöst innerhalb von 30 Minuten zugetropft. Es wird 75 Minuten bei + 35 °C Innentemperatur nachgerührt. Nach dem Abkühlen auf + 3 bis + 5 °C Innentemperatur rührt man 60 Minuten nach, saugt Magnesiumsulfat und Triethylaminhydrochlorid ab. Der Rückstand wird portionsweise mit insgesamt 110 ml Dimethylformamid nachgewaschen.

### B.) Kondensation der Schiff'schen Base der Glycinester mit N-[1-(4-Benzyloxy-indolyl-3-yl)-2-methoxyethyl]-N-isopropylamin (4-Benzyloxypseudogramin)

Das Filtrat A wird mit 51,0 g Kaliumcarbonat versetzt. Unter Erwärmen werden im Vakuum 110 ml Lösungsmittel abdestilliert. Unter schwachem Stickstoffstrom wird unter Normaldruck auf eine Innentemperatur von 90 -110 °C erwärmt. Hierzu wird eine Lösung von 50,0 g 4-Benzyloxypseudogramin in 295,0 ml Dimethylformamid innerhalb von 60 Minuten zugetropft. Bei + 100 - 110 °C Innentemperatur wird 90 Minuten nachgerührt. Nach dem Abkühlen auf Raumtemperatur wird der Feststoff abfiltriert und mit 110,0 ml Dimethylformamid nachgewaschen.

Das Filtrat wird bei einer Innentemperatur von + 20 - 25 °C mit 150,0 ml Wasser versetzt und mit ca. 52 ml halbkonzentrierter Salzsäure auf pH 2 angesäuert.

Innerhalb von 30 Minuten werden 11,02 ml 37 %ige wässrige Formaldehydlösung gelöst in 137,0 ml Wasser bei einer Innentemperatur von + 25 °C zugetropft. Nach zweistündigem Nachrühren läßt man bei Raumtemperatur über Nacht stehen.

Die Lösung wird mit 665,0 ml Wasser versetzt und mit 550 ml Toluol extrahiert. Anschließend fügt man 1100,0 ml Toluol hinzu, erwärmt auf + 30 bis 35 °C und versetzt portionsweise mit 41 g Natriumhydrogencarbonat. Die Toluolphase wird mit 550 ml Wasser gewaschen und über Natriumsulfat getrocknet. Das Filtrat wird im Vakuum bei einer Badtemperatur von + 40 °C auf ein Volumen von 665 ml eingeengt.

Die Toluollösung wird mit 45,3 ml Triethylamin versetzt und auf - 15 °C Innentemperatur gekühlt. Eine Lösung von 22,89 g Trichlorisocyanursäure in 635,0 ml Isopropylacetat wird eisgekühlt innerhalb von 20 Minuten zur Toluollösung getropft, dabei steigt die Innentemperatur auf - 8 °C. Nach 5 minütigem Nachrühren werden 55,7 ml Triethylamin zugesetzt und 3 Stunden bei Raumtemperatur gerührt. Nach dem Stehen über Nacht werden unter kräftigem Rühren 500 ml Wasser zugetropft. Nach 30 Minuten Rühren wird der Niederschlag abgesaugt, mit Wasser gewaschen und bei + 40 °C im Vakuum über Kaliumhydroxid getrocknet. Die organische Phase wird auf ein Volumen von 100 ml eingeengt, mit 2 ml Triethylamin versetzt und über Nacht zur Kristallisation abgestellt. Man erhält 41,8 g 5-Benzyloxy-4-methoxymethyl-β-carbolin-3- carbonsäureisopropylester vom Schmelzpunkt 209 °C ( 69,95 % der Theorie).

### Beispiel 2

Aus 50g N[1-(5-Benzyloxy-indolyl-3-yl)-2-methoxyethyl]-N-isopropylamin (5-Benzyloxypseudogramin) werden analog Beispiel 1 39,80 g (66,60 % der Theorie) vom Schmelzpunkt 150-151 °C 6-Benzyloxy-4-methoxymethyl-β-carbolin-3-carbonsäureisopropylester erhalten.

### Beispiel 3

Durch Umsetzen von 37,02 g N-[1-(4-Benzyloxyindol-3-yl)-ethyl]-N-isopropylamin analog Beispiel 1 erhält man 31,7 g (70,5 % der Theorie) 5-Benzyloxy-4-methyl-β-carbolin-3-carbonsäureisopropylester vom Schmelzpunkt 190-191 °C (aus Toluol-Methanol 1:1: kristallisiert).

### Beispiel 4

5,39 g N-{1-(4-[4-Chlorphenoxy]-indol-3-yl)-methoxymethyl}-N-isopropylamin analog Beispiel 1 umgesetzt liefert 3,28 g (51,4 % der Theorie) 5-(4-Chlorphenoxy)-4-methoxymethyl-β-carbolin-3-carbonsäureisopropylester vom Schmelzpunkt 119-120 °C.

### Beispiel 5

Aus 1,57 g N-{1-(4-[4-Chlorphenoxy]-indol-3-yl)-methyl}-N-isopropylamin gemäß Beispiel 1 werden 0,72 g 5-(4-Chlorphenoxy)-β-carbolin-3-carbonsäureisopropylester vom Schmelzpunkt 273-274 °C (Ausbeute 37,9 % der Theorie).

### Beispiel 6

Durch Kondensation von 2-(4-Methoxybenzylidenamino)-essigsäuremethylester und N-Isopropyl-N-[1(5,6-dimethoxyindol-3-yl)-propyl]-amin und weitere Umsetzung wie in Beispiel 1 beschrieben erhält man 6,7-Dimethoxy-4-ethyl-β-carbolin-3-carbonsäuremethylester vom Schmelzpunkt 207-209 °C (Ausbeute 52 % der Theorie).

## Patentansprüche

1. Verfahren zur Herstellung von Verbindungen der Formel I 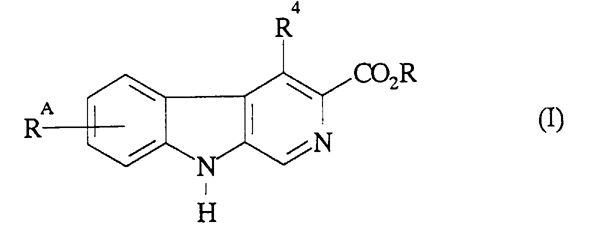 worin
R^{A} Wasserstoff, Halogen, -CHR¹-R², gegebenenfalls mit Halogen, C₁₋₄-Alkoxy oder Amino substituiertes Phenyl, OR⁵ oder einen 5- oder 6-gliedrigen Hetarylrest mit 1-3 Stickstoff-, Sauerstoff -und/oder Schwefelatomen bedeutet und ein- bis zweifach stehen kann und
R¹ Wasserstoff oder C₁₋₄-Alkyl,
R² Wasserstoff, C₁₋₄-Alkyl, -O-C₁₋₄-Alkyl oder einen gegebenenfalls ein- bis dreifach mit Halogen, Nitro, Cyano, C₁₋₄-Alkyl, C₁₋₄-Alkoxy, Amino, C₁₋₄-Alkoxycarbonyl, C₁₋₄-Alkylthio oder Trifluormethyl substituierten Phenyl-, Benzyl- oder Phenoxy-Rest,
R⁵ Wasserstoff, C₁₋₆-Alkyl, C₃₋₇-Cycloalkyl, einen gegebenenfalls ein- bis dreifach mit Halogen, Nitro, Cyano, C₁₋₄-Alkyl, C₁₋₄-Alkoxy, Amino, C₁₋₄-Alkoxycarbonyl, C₁₋₄-Alkylthio oder Trifluormethyl substituierten Phenyl-, Benzyl- oder 5- oder 6-gliedrigen Hetarylrest mit 1-3 Stickstoff-, Sauerstoff- und/oder Schwefelatomen oder einen gegebenenfalls ein bis dreifach mit Halogen, Nitro, Cyano, C₁₋₄-Alkyl, C₁₋₄-Alkoxy, Amino, C₁₋₄-Alkoxycarbonyl, C₁₋₄-Alkylthio oder Trifluormethyl substituierten benzokondensierten Hetarylrest mit 1-2 Stickstoffatomen bedeutet,
R⁴ Wasserstoff, C₁₋₄-Alkyl oder C₁₋₄-Alkoxy-C₁₋₂-alkyl und
R C₁₋₆-Alkyl bedeutet.,
dadurch gekennzeichnet, daß man eine Verbindung der Formel II 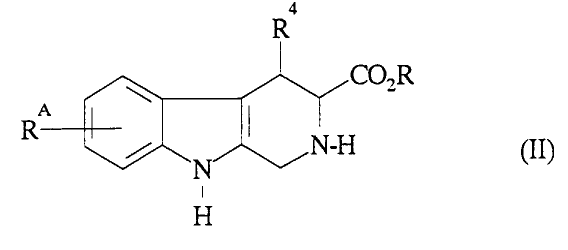 worin
R, R^{A} und R⁴ die obige Bedeutung haben mit Trichlorisocyanursäure in Gegenwart von Basen aromatisiert.

## Claims

1. Process for the preparation of compounds of formula I 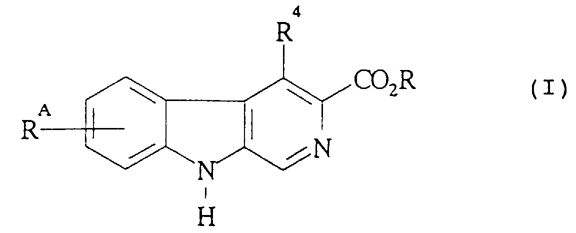 wherein
the radical R^{A}, of which there may be one or two, represents hydrogen, halogen, -CHR¹-R², optionally halo-, C₁₋₄alkoxy- or amino-substituted phenyl, OR⁵ or a 5- or 6-membered hetaryl radical having from 1 to 3 nitrogen, oxygen and/or sulphur atoms, and
R¹ represents hydrogen or C₁₋₄alkyl,
R² represents hydrogen, C₁₋₄alkyl, -O-C₁₋₄alkyl or a phenyl, benzyl or phenoxy radical that is optionally mono- to tri-substituted by halogen, nitro, cyano, C₁₋₄alkyl, C₁₋₄alkoxy, amino, C₁₋₄alkoxycarbonyl, C₁₋₄alkylthio or by trifluoromethyl,
R⁵ represents hydrogen, C₁₋₆alkyl, C₃₋₇cycloalkyl, a phenyl, benzyl or 5- or 6-membered hetaryl radical having from 1 to 3 nitrogen, oxygen and/or sulphur atoms that is optionally mono- to tri-substituted by halogen, nitro, cyano, C₁₋₄alkyl, C₁₋₄alkoxy, amino, C₁₋₄alkoxycarbonyl, C₁₋₄alkylthio or by trifluoromethyl or a benzo-condensed hetaryl radical having 1 or 2 nitrogen atoms that is optionally mono- to tri-substituted by halogen, nitro, cyano, C₁₋₄alkyl, C₁₋₄alkoxy, amino, C₁₋₄alkoxycarbonyl, C₁₋₄alkylthio or by trifluoromethyl,
R⁴ represents hydrogen, C₁₋₄alkyl or C₁₋₄alkoxy-C₁₋₂alkyl, and
R represents C₁₋₆alkyl,
characterised in that a compound of formula II 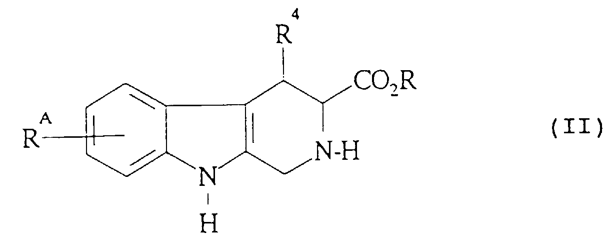 wherein
R, R^{A} and R⁴ have the meanings given above is aromatised with trichloroisocyanuric acid in the presence of bases.

## Revendications

1. Procédé pour préparer des composés de formule I 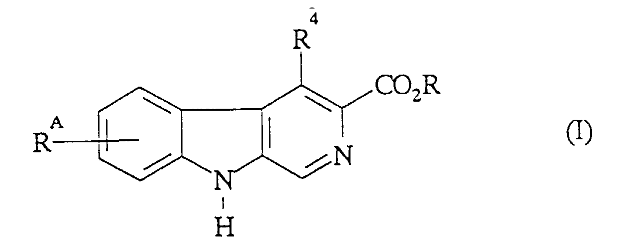 dans laquelle
R^{A} est un hydrogène, halogène, -CHR¹-R², phényle éventuellement substitué par des substituants halogéno, alcoxy en C1-4 ou amino, OR⁵ ou encore un radical hétéroaryle à 5 ou 6 chaînons ayant de 1 à 3 atomes d'azote, d'oxygène et/ou de soufre, et peut être présent une ou deux fois, et
R¹ est un hydrogène ou un radical alkyle en C₁₋₄,
R² est un hydrogène,ou un radical alkyle en C₁₋₄, -O-alkyle en C₁₋₄ ou un radical phényle, benzyle ou phénoxy, éventuellement une à trois fois substitué par des substituants halogéno, nitro, cyano, alkyle en C₁₋₄, alcoxy en C₁₋₄, amino, (alcoxy en C₁₋₄)carbonyle, alkylthio en C₁₋₄ ou trifluorométhyle,
R⁵ est un hydrogène, alkyle en C₁₋₆, cycloalkyle en C₃₋₇, un radical phényle, benzyle ou hétéroaryle à cinq ou six chaînons, éventuellement une à trois fois substitué par des substituants halogéno, nitro, cyano, alkyle en C₁₋₄, alcoxy en C₁₋₄, amino, (alcoxy en C₁₋₄)carbonyle, alkylthio en C₁₋₄ ou trifluorométhyle, le radical hétéroaryle ayant de 1 à 3 atomes d'azote, d'oxygène et/ou de soufre, ou encore représente un radical hétéroaryle benzo-condensé ayant 1-2 atomes de carbone, éventuellement une à trois fois substitué par des substituants halogéno, nitro, cyano, alkyle en C₁₋₄, alcoxy en C₁₋₄, amino, (alcoxy en C₁₋₄)carbonyle, alkylthio en C₁₋₄ ou triflurométhyle,
R⁴ est un hydrogène alkyle en C₁₋₄ ou (alcoxy en C₁₋₄)(alkyle en C₁₋₂), et
R est un radical alkyle en C₁₋₆,
caractérisé en ce qu'on aromatise un composé de formule II 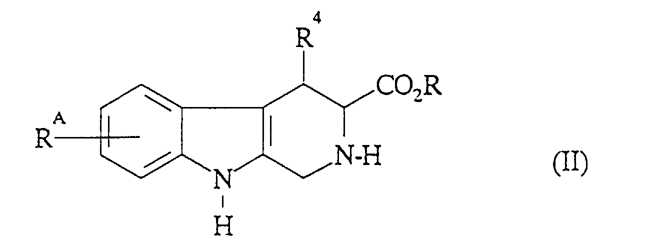 dans laquelle
R, R^{A} et R⁴ ont les significations données ci-dessus, avec de l'acide trichlorisocyanurique en présence de bases.
